# EUROPEAN PATENT SPECIFICATION

(11) **EP 0 536 250 B1**
(45) Date of publication and mention of the grant of the patent: **06.09.2000**
(21) Application number: 91912299.4
(22) Date of filing: 27.06.1991
(51) Int. Cl.: A61K 9/00, A61K 9/12

(54) **AEROSOL DRUG FORMULATIONS**
AEROSOL ARZNEIFORMULIERUNGEN
COMPOSITIONS DE MEDICAMENTS EN AEROSOL

(30) Priority: 28.06.1990 US 545437; 01.02.1991 US 649405
(43) Date of publication of application: 14.04.1993
(73) Proprietor: GLAXO WELLCOME INC., Research Triangle Park, North Carolina 27709 (US)
(72) Inventor: JOHNSON, Keith, A., Durham, NC 27707 (US)
(74) Representative: Filler, Wendy Anne, Dr.
(86) International application number: US9104715
(87) International publication number: WO9200107

(56) References cited:
- EP-A- 0 372 777
- WO-A-91/14422
- US-A- 4 352 789
- Pharmaceutical Technology, Vol. 26, March 1990, "CFC Propellant Substitution: P-134a as a Potential Replacement for P-12 in MDIs", DALBY et al., see entire document.

## Description

Drugs for treating respiratory and nasal disorders are frequently administered in aerosol formulations through the mouth or nose. Peter Byron, Respiratory Drug Delivery, CRC Press, Boca Raton, FL 1990, provides a background for this form of therapy. (As used hereinafter the terms "aerosol drug formulation" and "inhalation drug formulation" are synonymous and refer to one or more physiologically active chemical compounds in combination with excipients such as surface-active agents, "surfactants" and propellants.)

One widely used method for dispensing such an aerosol drug formulation involves making a suspension formulation of the drug as a finely divided powder in a liquefied gas known as a propellant. The suspension is stored in a sealed container capable of withstanding the pressure required to maintain the propellant as a liquid. The suspension is dispensed by activation of a dose metering valve affixed to the container. A metering valve may be designed to consistently release a fixed, predetermined amount of the drug formulation upon each activation. As the suspension is forced from the container through the dose metering valve by the high vapor pressure of the propellant, the propellant rapidly vaporizes leaving a fast moving cloud of very fine particles of the drug formulation. This cloud is usually directed into the body of the patient by a channeling device, e.g., a cylinder like or cone like passage, with one of its ends attached to the outlet of the pressurized container, and the other end inserted in the mouth or nose of the patient. Concurrently with the activation of the aerosol dose metering valve, the patient inhales the drug formulation particles into the lungs or nasal cavity. Systems for dispensing drugs in this way are known as "metered dose inhalers (MDI's)." [Ibid Byron, Pages 167-207.]

Many materials, including drug formulations, have a tendency to aggregate (also referred to as "flocculate" or "clump-up") when stored as fine particles having dimensions of a few microns in a suspension. For an aerosol delivery system to work properly the particle size should generally not exceed about five microns. As the particle size exceeds five microns, it becomes increasingly difficult to maintain an efficacious aerosol dose with a predicable dispersion pattern upon activation of the metering valve. Further, the suspension should be uniform, that is, substantially free from large aggregates of the drug particle and be substantially homogenous throughout the container.

To minimize or prevent the problem of aggregation of fine particles, compounds known as surface active agents, or surfactants, are used to coat the surfaces of the fine particles and assist in wetting the particles with an aerosol propellant. The use of surfactants in this way to maintain substantially uniform suspensions is said to "stabilize" the suspensions. An ideal surfactant should have a relative high affinity for the suspended drug formulation and be chemically and physically compatible with the propellant as well as the drug formulation. If it does not have these properties, the surfactant can possibly destabilize the suspension. Additionally, it must be essentially nontoxic.

For several years the chlorofluorocarbons (CFC's), for example, trichloromonofluoromethane, dichlorotetrafluoroethane and dichlorodifluoromethane, also known as "propellant 11" or "P 11", "propellant 114 or "P 114" and "propellant 12" or "P 12", respectively, have gained widespread acceptance as the propellants of choice for inhalation drug formulations. They are nonflammable, have low toxicity and reactivity, are compatible with many drug formulations and have the requisite physical attributes. See John Sciarra and Anthony Curie, Theory and Practice of Industrial Pharmacy, Pages 589-619, Lea and Febiger, Philadelphia, 1986. However, in the past few years CFC's have been shown to cause depletion of the ozone layer of the atmosphere, a serious environmental problem. Scientists and governmental officials around the world have called for a phase-out of the use of CFC's. Some countries, e.g., Sweden, have completely banned the use of CFC's for aerosol products, while other counties have levied substantial taxes on them to encourage the use of other, environmentally safer propellants. See Dalby, et al., Pharmaceutical Technology, 26, March 1990.

In recent years a nonchlorinated propellant chemically identified as 1,1,1,2-tetrafluoroethane also known as "propellant 134a" or "P 134a" has been promoted by major chemical manufacturers, notably DuPont and ICI, as an environmentally acceptable alternative to CFC propellants. Propellant 134a has physical properties comparable with P 12. Although like P 12 it is nonflammable and has a relatively low potential for interaction with a wide variety of products normally sold in aerosol form, its other chemical and solvent properties are different from P 12. For example P 134a is much less stable chemically than P 12 according to Dalby, et al. (see above).

Thiel in U.S. patent number 4,357,789 teaches the use of propellant insoluble perfluorinated surface-active dispersing agents in CFC and perfluorinated propellants although P 134a is not specifically mentioned. These agents include perfluorinated sulfonamide alcohol phosphate esters and their salts, perfluorinated alkyl sulfonamide alkylene quaternary ammonium salts and perfluorinated alcohol phosphate esters and their salts. Thiel teaches that surfactants must be insoluble in the propellant. Further, he teaches that the drug must be coated with the surfactant in an organic solvent dried, then added to the propellant mixture.

European Patent Application Publication No. 0 372 777 describes medicinal aerosol formulations which employ P134a as a propellant EPA 0 372 777 teaches that a four component system, comprising drug, surfactant, P134a and an adjuvant having higher polarity than P134a, is essential to obtain medicinal aerosol formulations having suitable properties for use with pressurised inhalers.

It has now been found that P 134a-soluble surfactants, especially soluble perfluorinated surfactants, effectively improve the stability of micronized inhalation drug suspensions in P 134a. (As used herein the terms "perfluorinated" and "perfluoro" mean that for at least one alkyl group essentially all of the hydrogens are substituted with fluorine.) Accordingly, when a micronized drug, i.e., a drug having an average particle size of about 5 microns or below and a maximum particle size of less than about 10 microns, and a P 134a soluble surfactant are placed in P 134a in a pressurized container, there is considerably less tendency for the drug particles to aggregate and separate from the suspension than the drug formulation without such surfactant or with a P 134a hydrocarbon surfactant commonly used with a CFC propellant. Thus it is now possible with the present invention to prepare aerosol formulations of inhalation drugs with P 134a which have sufficient stability for the purposes of this invention to deliver the active drug in the desired way as presently marketed MDI's, but without the environmental problems associated with CFC's. As used herein the term "sufficient stability" means that the aerosol drug formulation remains as a suspension after shaking at least long enough to allow activation of the MDI and administration by the patient. The time between shaking and administration is typically about 10 sec. and generally for the formulations of this invention the period of stability is at least about 30 sec.

The present invention provides an aerosol formulation as set out in claim 1 appended hereto.

An aspect of this invention is the use of one or more P 134a soluble surfactants to stabilize an inhalation drug in P 134a. A second aspect is an aerosol inhalation drug formulation comprising a physiologically effective amount of a micronized inhalation drug and one or more P 134a soluble surfactants in suspension in P 134a.

In a preferred aspect the invention provides an aerosol drug formulation comprising a physiologically effective amount of micronized inhalation drug and one or more P134a soluble surfactants in suspension in P134a which formulation is substantially free of P134a insoluble surfactant.

In a further or alternative aspect the invention provides an aerosol drug formulation comprising a particulate drug and one or more P134a soluble surfactants in suspension in P134a, which formulation is substantially free of drug which has been coated with surfactant prior to addition to the propellant mixture.

In a further or alternative aspect there is also provided an aerosol drug formulation comprising a medicament, P134a and one or more P134a soluble surfactants which formulation is substantially free of an adjuvent having a higher polarity than P134a. It will be appreciated by those skilled in the art that such a formulation, which is essentially a three component formulation may also contain other excipients normally included in medicinal aerosol formulations.

Especially useful drugs include respiratory drugs such as β₂-stimulants (β₂-agonists), anticholinergic drugs, corticosteroids and antiallergic drugs.

β₂-stimulants include for example fenoterol, pirbuterol, reproterol, imiterol, terbutalline, tulobuterol, isoprenaline and oxaprenaline.

Anticholinergic drugs include ipratropium bromide and oxitropium bromide. Corticosteroids include budesonide.

Antiallergic drugs include sodium cromoglycate, ketotifen and nedocromil sodium.

Of particular use in this invention are the respiratory drugs albuterol, salmeterol, amiloride, fluticasone propionate, beclomethasone dipropionate and (-)-4-amino-3,5-dichloro-α-[[[6-[2-(2-pyridinyl)ethoxy]hexyl]amino]methyl]benzenemethanol.

United States Patent number 3,644,353 teaches a group of bronchodilating compounds that are particularly useful in the treatment of asthma and other respiratory diseases. The preferred compound taught therein is α¹-tert-butylaminomethyl-4-hydroxy-m-xylene-α¹, α³-diol, also known in the United States by its generic name, "albuterol" and, in most other countries, "salbutamol." This compound, especially in aerosol form, has been widely accepted by the medical community in the treatment of asthma.

Salmeterol, chemically named 4-hydroxy-α'-[[[6[(4-phenylbutyl)oxy]hexyl]amino]methyl]-1,3-benzenedimethanol, disclosed in British Patent Publication No. 2140800 is a second generation bronchodilator which is longer acting and more potent than albuterol. This compound is in not yet marketed in the United States, but clinical trials in other countries indicate that a preferred mode of administration is by way of aerosol inhalation.

The genetic disease cystic fibrosis is characterized by abnormalities that produce excessive pulmonary secretion which can make breathing difficult. United States Patent Number 4,501,729 discloses the use of the drug amiloride in an aerosol formulation to reduce the excess secretion.

United Kingdom Patent Specification No. 2088877 discloses fluticasone esters. Fluticasone esters are corticosteroids having topical anti-inflammatory action. Corticosteroids may be used in the management of patients whose asthma is inadequately treated by bronchodilators and/or sodium cromoglycate.

A further class of corticosteroids having topical anti-inflammatory action, beclomethasone esters, are described in United Kingdom Patent Specification No. 1 047 519.

(-)-4-Amino-3,5-dichloro-α-[[[6-[2-(2-pyridinyl)ethoxy]hexyl] amino]methyl]benzenemethanol is a bronchodilator.

Where appropriate the drugs may be used in the form of salts (e.g. as alkali metal or amine salts or as acid addition salts) or as esters (e.g. as lower alkyl esters).

For use in the invention, albuterol will preferably be in the form of the sulphate salt or the free base and salmeterol will preferably be in the form of its 1-hydroxy-2-naphthoate salt. The preferred fluticasone ester for use in the invention is fluticasone propionate, and the preferred beclomethasone ester is beclomethasone dipropionate.

In addition to surfactants it may be desirable to add other excipients to an aerosol formulation to improve drug delivery, shelf life and patient acceptance. Such optional excipients include, but are not limited to, coloring agents, taste masking agents, buffers, antioxidants and chemical stabilizers.

Inhalation drugs, or a pharmaceutically acceptable salt hereof, may be micronized by, for example, conventional jet mill micronizing to particles ranging from 0.1 to 10.0 microns and preferably from 0.5 to 5.0 microns. The micronized inhalation drug or combination of drugs are mixed with one or more P 134a-soluble surfactants and, optionally, other excipients and then placed in a suitable container capable of withstanding the vapor pressure of P 134a and fitted with a metering valve. The propellant is then forced as a liquid through the valve into the container. The completed MDI is shaken vigorously to form the suspension.

Alternatively, an MDI can also be produced by adding drug, surfactant and liquefied propellant 134a (chilled below it's boiling point) to the container and then a metering valve fitted to the container. The completed MDI can then be brought to ambient temperature and shaken vigorously to form the suspension.

MDI's prepared according to the teachings herein may be used in the same way as currently marketed MDI's which use CFC's or hydrocarbon propellants. For example, in the case of albuterol, amount of drug, surfactant and propellant can be adjusted to deliver 90 µg per valve actuation, the dose delivered in currently marketed albuterol MDI's.

Particular P 134a-soluble surfactants include perfluorinated surfactants, especially perfluoroalkanoic acid surfactants having greater than 4 but 20 or less carbons, preferably from 8 to 10 carbons. Also particularly suitable are a mixture of potassium perfluoroalkyl sulfonates and a mixture of ammonium perfluoroalkyl carboxylates available under the trademarks FC-95 and FC-143, respectively, from 3M Corporation, Saint Paul, Minn. Most suitable are the perfluoroalkanoic acids, perfluorooctanoic acid and perfluorodecanoic acid.

The ratio of surfactant to drug is from 1:100 to 1:0.5 by weight, preferably in the range of 1:50 to 1:1 and most preferably in the range of 1:25 to 1:1 by weight. The amount of P 134a can be varied according to the amount of drug formulation to be delivered with each activation of the dose metering valve. Typically for an inhalation drug the amount of P 134a for each formulation of active drug depends on the volume of the metering valve and the dose desired. However the ratio of active drug or drugs to P 134a is in the range from 1:100 to 1:4000 by weight. For example, for albuterol in an aerosol inhalation system outfitted with a Bespak BK300 valve, 18 g of P 134a are utilized per 50 mg of albuterol to deliver an effective dose of albuterol.

The instant invention thus provides an aerosol inhalation drug formulation comprising a physiologically effective amount of a micronized drug suitable for inhalation therapy and a propellant 134a-soluble surfactant in suspension in propellant 134a and optionally other excipients.

The following examples are presented for illustration of the invention and are not to be construed as a limitation thereto.

### EXAMPLES

### General Procedure

Micronized drug and surfactant (if used) are weighed into a 15 mL transparent aerosol vial (No. S-24F6, produced by Wheaton Industries, NJ). A metering valve (Bespak valve No. BK300 produced by Bespak plc, England) is crimped onto each vial. Finally, Propellant 134a (from E. I. DuPont de Nemours and Company, Wilmington Del.) is added to the vial through the valve. Vials are then vigorously shaken for 30 mm with a wrist-action shaker.

Immediately after shaking, the suspension in the transparent vial is very milky or turbid. If left undisturbed, the drug particles eventually flocculate and concentrate at the gas/liquid interface (creaming) or at the bottom of the vial (settling) leaving behind a relatively clear Propellant 134a region. By shaking a formulation that has separated, it quickly re-disperses to a milky suspension. Suspension stability is assessed by monitoring the rate at which the drug particles flocculates as evidenced by the time required for the suspension to become coarse and/or to develop a relatively clear propellant region. If significant flocculation occurs, that is, a cognizable coarseness and/or clear region can be observed, in less than about 15 sec., the suspension is deemed not stable enough for a practical aerosol inhalation drug formulation.

Alternatively, several suspensions can be shaken simultaneously and the most stable suspension designated as the last one to separate. A suspension of drug in Propellant 134a with no surfactant is used as a control and reference for measuring the stability of the formulations.

The drug and propellant weight ratio is selected based on reasonable ranges of marketed products. The ratio of surfactant weight to drug weight is varied by keeping the drug weight constant and increasing the surfactant weight.

Using the procedure described above, the following data shown on Table 1 below was obtained:

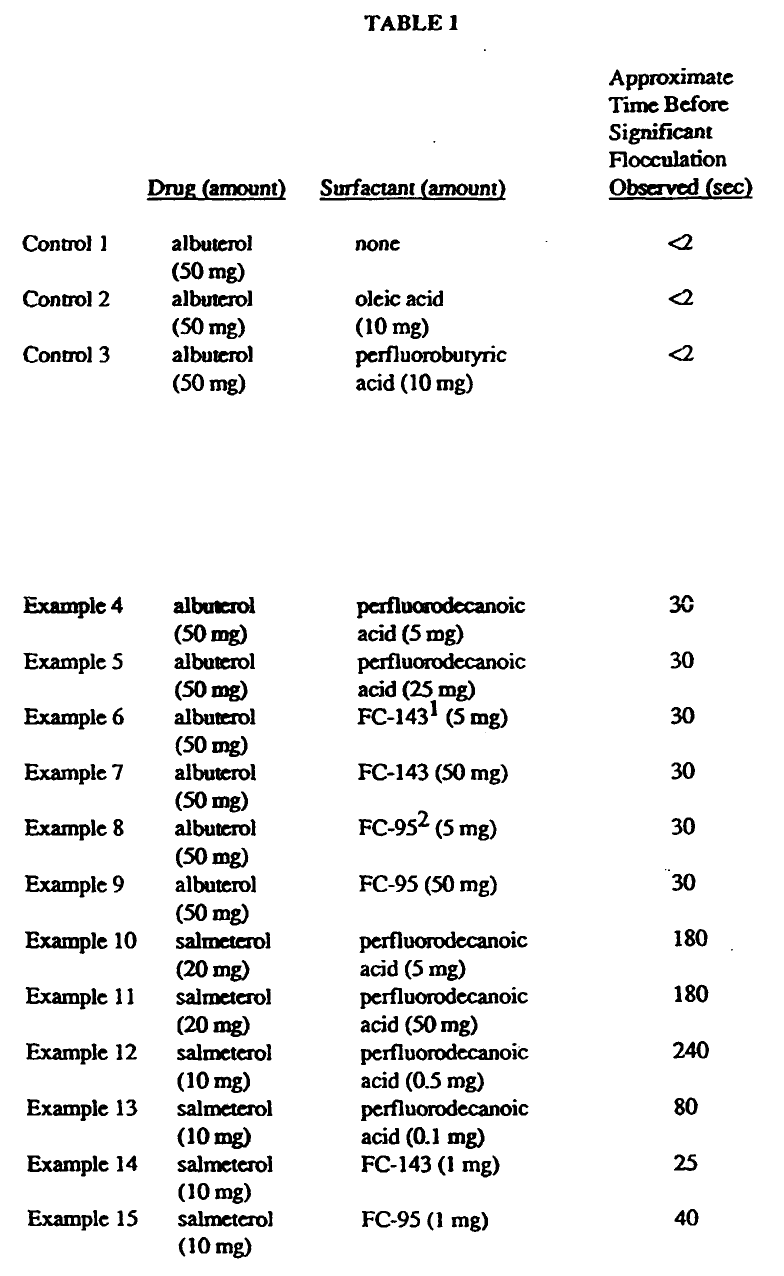

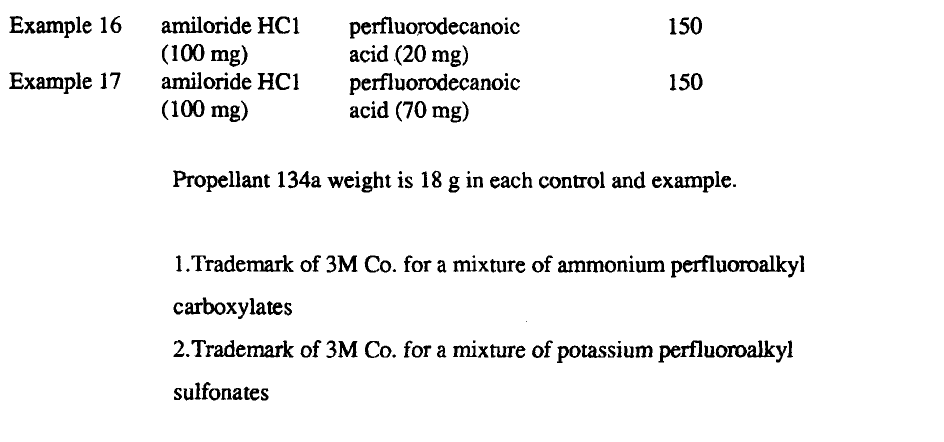

In the case of albuterol, the rate of particle settling after flocculation tended to increase with surfactant concentration.

The following are examples of stable micronized drug suspension formulations according to the invention.

### Example 18

Chilled propellant 134 a (18g) was added to perfluorodecanoic acid (25mg) in a glass aerosol vial. Micronised Beclomethansone dipropionate (50mg) was added and a metering valve crimped into place. The process was performed in a dry box.

### Example 19

Chilled propellant 134a (18g) was added to perfluorodecanoic acid (50mg) in a glass aerosol vial. Micronised Beclomethanasone dipropionate (50mg) was added and a metering valve crimped into place. The process was performed in a dry box.

### Example 20

Chilled propellant 134a (18g) was added to perfluorodecanoic acid (1mg) in a glass aerosol vial. Micronised Albuterol sulphate (32mg) was added and a metering valve crimped into place. The process was performed in a dry box.

### Example 21

Micronised fluticasone propionate (50mg) and perfluorodecanoic acid (20mg) were weigh into a glass aerosol vial. A metering valve was crimped onto the vial and propellant 134a (18g) added to the vial throught the valve.

### Example 22

Micronised fluticasone propionate (50mg) and perfluorodecanoic acid (50mg) were weigh into a glass aerosol vial. A metering valve was crimped onto the vial and propellant 134a (18g) added to the vial throught the valve.

## Claims

1. An aerosol formulation comprising a particulate inhalation drug having a maximum particle size of less than 10 microns, 1,1,1,2-tetrafluoroethane as propellant, and a perfluorinated surfactant soluble in 1,1,1,2-tetrafluoroethane, which formulation contains essentially no adjuvant having a higher polarity than 1,1,1,2-tetrafluoroethane, with the proviso that when said surfactant is a perfluorinated aliphatic carboxylic acid CₙF₂ₙ₊₁COOH wherein n is an integer from 3 to 10, said surfactant is present in an amount other than 0.001% to 0.6% by weight based on the total weight of the formulation.

2. An aerosol formulation consisting of a particulate inhalation drug having a maximum particle size of less than 10 microns, 1,1,1,2-tetrafluoroethane as propellant, and a perfluorinated surfactant soluble in 1,1,1,2-tetrafluoroethane, with the proviso that when said surfactant is a perfluorinated aliphatic carboxylic acid CₙF₂ₙ₊₁COOH wherein n is an integer from 3 to 10, said surfactant is present in an amount other than 0.001% to 0.6% by weight based on the total weight of the formulation.

3. A formulation as claimed in claim 1 or claim 2 wherein the ratio of said surfactant to said drug is from 1:100 to 1:0.5 by weight.

4. A formulation as claimed in any one of claims 1 to 3 wherein the ratio of said drug to 1,1,1,2-tetrafluoroethane is from 1:100 to 1:4000 by weight.

5. A formulation as claimed in any one of claims 1 to 4 wherein said surfactant is a perfluoroalkanoic acid of greater than 4 carbons but not greater than 20 carbons.

6. A formulation as claimed in claim 5 wherein said perfluoroalkanoic acid is perfluorodecanoic acid.

7. A formulation as claimed in any one of claims 1 to 6 wherein the inhalation drug is an anti-allergic, β₂-stimulant, anticholinergic or corticosteroid.

8. A formulation as claimed in any one of claims 1 to 6 wherein said inhalation drug is salbutamol or a pharmaceutically acceptable salt thereof.

9. A formulation as claimed in claim 8 wherein the salbutamol is in the form of the sulphate salt.

10. A formulation as claimed in claim 8 wherein the salbutamol is in the form of the free base.

11. A formulation as claimed in any one of claims 1 to 6 wherein said inhalation drug is salmeterol or a pharmaceutically acceptable salt thereof.

12. A formulation as claimed in claim 11 wherein the salmeterol is in the form of its 1-hydroxy-2-naphthoate salt.

13. A formulation as claimed in any one of claims 1 to 6 wherein said inhalation drug is beclomethasone dipropionate.

14. A formulation as claimed in any one of claims 1 to 6 wherein said inhalation drug is fluticasone propionate.

15. A formulation as claimed in any one of claims 1 to 6 wherein the inhalation drug is amiloride.

16. A formulation as claimed in any one of claims 1 to 6 wherein the inhalation drug is (-)-4-amino-3,5-dichloro-α-[[[6-[2-(2-pyridinyl)ethoxy]hexyl]amino] methyl]benzenemethanol.

17. A formulation as claimed in any one of claims 1 to 6 wherein the inhalation drug is budesonide.

18. A formulation as claimed in any one of claims 1 to 6 wherein the inhalation drug is selected from fenoterol, reproterol, imiterol, terbutalline, tulobuterol, isoprenaline, oxiprenaline, ipratropium bromide, oxitropium bromide and ketotifen.

19. An aerosol formulation comprising a particulate inhalation drug selected from the group salbutamol, salmeterol and amiloride or a pharmaceutically acceptable salt thereof having a maximum particle size of less than 10 microns and a 1,1,1,2-tetrafluoroethane-soluble perfluorinated surfactant in suspension in 1,1,1 ,2-tetrafluoroethane propellant wherein the ratio of said surfactant to said drug is from 1:100 to 1:0.5 by weight and the ratio of drug to 1,1,1,2-tetrafluoroethane is from 1:100 to 1:4000 by weight and optionally other excipients, which formulation contains essentially no adjuvant having a higher polarity than 1,1,1,2-tetrafluoroethane, with the proviso that when said surfactant is a perfluorinated aliphatic carboxylic acid CₙF₂ₙ₊₁COOH wherein n is an integer from 3 to 10, said surfactant is present in an amount other than 0.001% to 0.6% by weight based on the total weight of the formulation.

20. A formulation as claimed in any one of claims 1 to 19 wherein said surfactant is potassium perfluoroalkyl sulphonate or ammonium perfluoroalkyl carboxylate or a combination thereof.

21. A formulation as claimed in claim 19 wherein said surfactant is perfluorodecanoic acid.

## Patentansprüche

1. Aerosol-Formulierung, umfassend einen teilchenförmigen Inhalationsarzneistoff mit einer maximalen Teilchengröße von weniger als 10 µm, 1,1,1,2-Tetrafluorethan als Treibmittel und ein in 1,1,1,2-Tetrafluorethan lösliches perfluoriertes Tensid, wobei die Formulierung im wesentlichen keinen Hilfsstoff mit einer höheren Polarität als 1,1,1,2-Tetrafluorethan enthält, mit der Maßgabe, daß das Tensid in einer von 0,001 bis 0,6 Gew.-% verschiedenen Menge, bezogen auf das Gesamtgewicht der Formulierung, vorhanden ist, wenn das Tensid eine perfluorierte aliphatische Carbonsäure CₙF₂ₙ₊₁COOH ist, worin n eine ganze Zahl von 3 bis 10 ist.

2. Aerosol-Formulierung, bestehend aus einem teilchenförmigen Inhalationsarzneistoff mit einer maximalen Teilchengröße von weniger als 10 µm, 1,1,1,2-Tetrafluorethan als Treibmittel und einem in 1,1,1,2-Tetrafluorethan löslichen perfluorierten Tensid, mit der Maßgabe, daß das Tensid in einer von 0,001 bis 0,6 Gew.-% verschiedenen Menge, bezogen auf das Gesamtgewicht der Formulierung, vorhanden ist, wenn das Tensid eine perfluorierte aliphatische Carbonsäure CₙF₂ₙ₊₁COOH ist, worin n eine ganze Zahl von 3 bis 10 ist.

3. Formulierung gemäß Anspruch 1 oder 2, worin das Gewichtsverhältnis des Tensids zum Arzneistoff 1:100 bis 1:0,5 beträgt.

4. Formulierung gemäß einem der Ansprüche 1 bis 3, worin das Gewichtsverhältnis des Arzneistoffs zu 1,1,1,2-Tetrafluorethan 1:100 bis 1:4000 beträgt.

5. Formulierung gemäß einem der Ansprüche 1 bis 4, worin das Tensid eine Perfluoralkansäure mit mehr als 4 Kohlenstoffatomen aber nicht mehr als 20 Kohlenstoffatomen ist.

6. Formulierung gemäß Anspruch 5, worin die Perfluoralkansäure Perfluordecansäure ist.

7. Formulierung gemäß einem der Ansprüche 1 bis 6, worin der Inhalationsarzneistoff ein Antiallergikum, α₂-Stimulans, Anticholinergikum oder Kortikosteroid ist.

8. Formulierung gemäß einem der Ansprüche 1 bis 6, worin der Inhalationsarzneistoff Salbutamol oder ein pharmazeutisch akzeptables Salz davon ist.

9. Formulierung gemäß Anspruch 8, worin das Salbutamol in Form des Sulfatsalzes ist.

10. Formulierung gemäß Anspruch 8, worin das Salbutamol in Form der freien Base ist.

11. Formulierung gemäß einem der Ansprüche 1 bis 6, worin der Inhalationsarzneistoff Salmeterol oder ein pharmazeutisch akzeptables Salz davon ist.

12. Formulierung gemäß Anspruch 11, worin das Salmeterol in Form seines 1-Hydroxy-2-naphthoatsalzes ist.

13. Formulierung gemäß einem der Ansprüche 1 bis 6, worin der Inhalationsarzneistoff Beclomethasondipropionat ist.

14. Formulierung gemäß einem der Ansprüche 1 bis 6, worin der Inhalationsarzneistoff Fluticasonpropionat ist.

15. Formulierung gemäß einem der Ansprüche 1 bis 6, worin der Inhalationsarzneistoff Amilorid ist.

16. Formulierung gemäß einem der Ansprüche 1 bis 6, worin der Inha-lationsarzneistoff (-)-4-Amino-3,5-dichlor-α-[[[6-[2-(2-pyridinyl)ethoxy]-hexyl]amino]methyl]benzolmethanol ist.

17. Formulierung gemäß einem der Ansprüche 1 bis 6, worin der Inha-lationsarzneistoff Budesonid ist.

18. Formulierung gemäß einem der Ansprüche 1 bis 6, worin der Inha-lationsarzneistoff ausgewählt ist aus Fenoterol, Reproterol, Imiterol, Terbutallin, Tulobuterol, Isoprenalin, Oxiprenalin, Ipratropiumbromid, Oxitropiumbromid und Ketotifen ist.

19. Aerosol-Formulierung, umfassend einen teilchenförmigen Inhalationsarzneistoff, ausgewählt aus der Gruppe Salbutamol, Salmeterol und Amilorid oder einem pharmazeutisch akzeptablen Salz davon, mit einer maximalen Teilchengröße von weniger als 10 µm und ein 1,1,1,2-Tetrafluorethan-lösliches perfluoriertes Tensid in Suspension in 1,1,1,2-Tetrafluorethan-Treibmittel, worin das Gewichtsverhältnis des Tensids zum Arzneistoff 1:100 bis 1:0,5 beträgt und das Gewichtsverhältnis von Arzneistoff zu 1,1,1,2-Tetrafluorethan 1:100 bis 1:4000 beträgt, und gegebenenfalls andere Arzneimittelzusatzstoffe, wobei die Formulierung im wesentlichen keinen Hiltsstoff mit einer höheren Polarität als 1,1,1,2-Tetrafluorethan enthält, mit der Maßgabe, daß das Tensid in einer von 0,001 bis 0,6 Gew.-% verschiedenen Menge, bezogen auf das Gesamtgewicht der Formulierung, vorhanden ist, wenn das Tensid eine perfluorierte aliphatische Carbonsäure CₙF₂ₙ₊₁COOH ist, worin n eine ganze Zahl von 3 bis 10 ist.

20. Formulierung gemäß einem der Ansprüche 1 bis 19, worin das Tensid Kaliumperfluoralkylsulfonat oder Ammoniumperfluoralkylcarboxylat oder eine Kombination daraus ist.

21. Formulierung gemäß Anspruch 19, worin das Tensid Perfluordecansäure ist.

## Revendications

1. Formulation en aérosol comprenant un médicament d'inhalation sous forme de particules, présentant une taille maximale des particules inférieure à 10 micromètres, du 1,1,1,2-tétrafluoroéthane comme gaz propulseur et un surfactant perfluoré soluble dans du 1,1,1,2-tétrafluoroéthane, ladite formulation ne contenant essentiellement pas d'adjuvant présentant une polarité supérieure au 1,1,1,2-tétrafluoroéthane, à condition que, lorsque ledit surfactant est un acide carboxylique aliphatique perfluoré CₙF₂ₙ₊₁COOH, où n est un nombre entier de 3 à 10, ledit surfactant soit présent en une quantité autre que de 0,001 % à 0,6 % en poids, sur base du poids total de la formulation.

2. Formulation en aérosol constituée par un médicament d'inhalation sous forme de particules, présentant une taille maximale des particules inférieure à 10 micromètres, du 1,1,1,2-tétrafluoroéthane comme gaz propulseur et un surfactant perfluoré soluble dans du 1,1,1,2-tétrafluoroéthane, à condition que, lorsque ledit surfactant est un acide carboxylique aliphatique perfluoré CₙF₂ₙ₊₁COOH, où n est un nombre entier de 3 à 10, ledit surfactant soit présent en une quantité autre que de 0,001 % à 0,6 % en poids, sur base du poids total de la formulation.

3. Formulation selon la revendication 1 ou la revendication 2, dans laquelle le rapport dudit surfactant audit médicament est de 1:100 à 1:0,5 en poids.

4. Formulation selon l'une quelconque des revendications 1 à 3, dans laquelle le rapport dudit médicament au 1,1,1,2-tétrafluoroéthane est de 1:100 à 1:4000 en poids.

5. Formulation selon l'une quelconque des revendications 1 à 4, dans laquelle ledit surfactant est un acide perfluoroalcanoïque comprenant plus de 4 atomes de carbone, mais moins de 20 atomes de carbone.

6. Formulation selon la revendication 5, dans laquelle ledit acide perfluoroalcanoïque est l'acide perfluorodécanoïque.

7. Formulation selon l'une quelconque des revendications 1 à 6, dans laquelle le médicament d'inhalation est un antiallergique, un β₂-stimulant, un anticholinergique ou un corticostéroïde.

8. Formulation selon l'une quelconque des revendications 1 à 6, dans laquelle ledit médicament d'inhalation est le salbutamol ou un sel pharmaceutiquement acceptable de celui-ci.

9. Formulation selon la revendication 8, dans laquelle le salbutamol est sous forme du sel de sulfate.

10. Formulation selon la revendication 8, dans laquelle le salbutamol est sous forme de la base libre.

11. Formulation selon l'une quelconque des revendications 1 à 6, dans laquelle ledit médicament d'inhalation est du salmétérol ou un sel pharmaceutiquement acceptable de celui-ci.

12. Formulation selon la revendication 11, dans laquelle le salmétérol est sous forme de son sel de 1-hydroxy-2-naphtoate.

13. Formulation selon l'une quelconque des revendications 1 à 6, dans laquelle ledit médicament d'inhalation est le dipropionate de béclométhasone.

14. Formulation selon l'une quelconque des revendications 1 à 6, dans laquelle ledit médicament d'inhalation est le propionate de fluticasone.

15. Formulation selon l'une quelconque des revendications 1 à 6, dans laquelle le médicament d'inhalation est l'amiloride.

16. Formulation selon l'une quelconque des revendications 1 à 6, dans laquelle le médicament d'inhalation est le (-)-4-amino-3,5-dichloro-α-[[[6-[2-(2-pyridinyl)éthoxy]hexyl]amino]méthyl]benzèneméthanol.

17. Formulation selon l'une quelconque des revendications 1 à 6, dans laquelle le médicament d'inhalation est du budésonide.

18. Formulation selon l'une quelconque des revendications 1 à 6, dans laquelle le médicament d'inhalation est choisi parmi le fénotérol, le réprotérol, l'imitérol, la terbutaline, le tulobutérol, l'isoprénaline, l'oxiprénaline, le bromure d'ipratropium, le bromure d'oxitropium et le kétotifen.

19. Formulation en aérosol comprenant un médicament d'inhalation sous forme de particules, choisi dans le groupe constitué par le salbutamol, le salmétérol et l'amiloride ou un sel pharmaceutiquement acceptable de ceux-ci, présentant une taille maximale de particules inférieure à 10 micromètres, et un surfactant perfluoré soluble dans du 1,1,1,2-tétrafluoroéthane, en suspension dans du gaz propulseur 1,1,1,2-tétrafluoroéthane, dans laquelle le rapport dudit surfactant audit médicament est de 1:100 à 1:0,5 en poids et le rapport du médicament au 1,1,1,2-tétrafluoroéthane est de 1:100 à 1:4000 en poids et, facultativement, d'autres excipients, ladite formulation ne contenant essentiellement pas d'autre adjuvant présentant une polarité supérieure au 1,1,1,2-tétrafluoroéthane, à condition que, lorsque ledit surfactant est un acide carboxylique aliphatique perfluoré CₙF₂ₙ₊₁COOH, où n est un nombre entier de 3 à 10, ledit surfactant soit présent en une quantité autre que de 0,001 % à 0,6 % en poids, sur base du poids total de la formulation.

20. Formulation selon l'une quelconque des revendications 1 à 19, dans laquelle ledit surfactant est un perfluoroalkylsulfonate de potassium ou un perfluoroalkylcarboxylate d'ammonium ou une combinaison de ceux-ci.

21. Formulation selon la revendication 19, dans laquelle ledit surfactant est l'acide perfluorodécanoïque.
